(19) Europäisches Patentamt

European Patent Office

Office européen des brevets

(11) Veröffentlichungsnummer: **0 520 201 A1**

# (12) EUROPÄISCHE PATENTANMELDUNG

(21) Anmeldenummer: **92109021.3**

(22) Anmeldetag: **29.05.92**

(51) Int. Cl.5: **A61M 16/00**, F04B 51/00, G01F 3/16, G01D 5/24, F15B 15/28

(30) Priorität: **25.06.91 DE 4120913**

(43) Veröffentlichungstag der Anmeldung: **30.12.92 Patentblatt 92/53**

(84) Benannte Vertragsstaaten: **DE FR GB SE**

(71) Anmelder: **Drägerwerk Aktiengesellschaft Moislinger Allee 53-55**

W-2400 Lübeck 1(DE)

(72) Erfinder: **Dittmann, Ralf**
**Junoring 7**
**W-2400 Lübeck 1(DE)**
Erfinder: **Dautzenberg, Brigitte**
**Lindenstrasse 41**
**W-2400 Lübeck(DE)**

(54) **Kolben-Zylindereinheit zur Atemgasförderung als Zylinderkondensator für die Hubstreckenmessung.**

(57) Eine Kolbenzylindereinheit als Fördervorrichtung für das Atemgas in einem Beatmungsgerät mit einer zwischen dem Kolben und dem Zylinder abrollenden Rollmembran soll so verbessert werden, daß zur Wegerfassung der Hubstrecke eine einfache, genau und reproduzierbar sowie absolut messende und ohne Verschleißteile auskommende Meßvorrichtung für die sich bei jedem Kolbenhub verändernden Hubstrecke möglich ist. Dazu ist vorgesehen, daß zumindest ein Teil der Zylinderwand (29) und der Kolbenwand (28) als elektrisch-leitfähige Platten eines Zylinder-Kondensators ausgebildet sind, und daß die Rollmembran (9) als Dielektrikum die sich jeweils überlappenden Wandteile von Kolben (4) und Zylinder (1) abdeckt. Die bei der Hubbewegung sich verändernde Kapazität des Kondensators wird zur Wegmessung für die Hubstrecke herangezogen.

FIG 1

Die Erfindung betrifft eine Kolben-Zylindereinheit als über eine Antriebseinheit ansteuerbare Fördervorrichtung für das Atemgas in einem Beatmungsgerät, wobei der Kolben relativ zu dem Zylinder bewegbar, seine Bewegung von einem Wegaufnehmer erfaßbar und eine zwischen Kolbenwand und Zylinderwand abrollende Rollmembran als Dichtung vorgesehen ist.

Eine derartige Fördervorrichtung ist aus der DE-OS 38 17 092 bekanntgeworden.

Bei der bekannten Kolben-Zylindereinheit wird der Kolben über eine Kolbenstange durch einen Antriebsmotor hin und her bewegt und mittels einer Doppelrollmembran entlang der Zylinderwand geführt, wobei die Position des Kolbens relativ zur Zylinderwand durch eine Lichtschranke abgetastet wird. Derartige Wegaufnehmer arbeiten als Inkrementalgeber, welche jedoch nur die relative Bewegung und die Bewegungsrichtung erfassen (vorwärts, rückwärts), jedoch nicht die absolute Position von einer Endlage aus gesehen.

Zur absoluten Wegmessung werden zwar Dreh- oder Schiebepotentiometer eingesetzt, die jedoch wegen der mechanischen Verschleißerscheinungen (Abrieb) nicht langzeitstabil genug sind, um eine auf Dauer genaue Wegmessung ermöglichen zu können. Außerdem liefern diese Potentiometer analoge Signale, die bei dem heutigen Einsatz digitaler Meßtechnik zur Weiterverarbeitung einen Analog-Digitalwandler erfordern.

Der vorliegenden Erfindung liegt somit die Aufgabe zugrunde, die Wegerfassung für eine Kolbenzylindereinheit so zu verbessern, daß eine einfache, genau und reproduzierbar, absolut messende und ohne Verschleißteile auskommende Meßvorrichtung für die sich bei jedem Kolbenhub verändernde Hubstrecke möglich wird.

Die Lösung der Aufgabe erfolgt dadurch, daß zumindest ein Teil der Zylinderwand einerseits und der Kolbenwand andererseits als Platten eines Zylinder-Kondensators ausgebildet sind, zwischen denen die Rollmembran als Dielektrikum die sich jeweils überlappenden elektrisch-leitenden Wandteile von Kolben und Zylinder abdeckt, und daß die bei der Hubbewegung sich verändernde Kapazität des Kondensators als Wegmessung für die Hubstrecke durch eine Auswerteeinheit erfaßbar ist.

Der Vorteil der Erfindung liegt im wesentlichen darin, daß eine mechanisch einfach aufgebaute, ohne zusätzliche Verschleißteile auskommende und absolut messende Meßvorrichtung gewonnen wird. Durch die Ausbildung der Kolbenzylindereinheit als kapazitiver Meßgeber kann die Auswertung des Meßsignals in Form einer einfachen Oszillator- oder Brückenschaltung verwirklicht werden, die eine direkte Ankopplung des Meßsignals an digital arbeitende elektronische Komponenten zur Weiterverarbeitung erlaubt. Kolbenwand und Zylinderwand sind teilweise oder ganz metallisch ausgeführt bzw. metallisch ausgekleidet oder beschichtet. Während einer Hubbewegung verändert sich die Kapazität C des Kondensators nach der bekannten Gesetzmäßigkeit

$$C = e_o \cdot e_r \cdot 2 \cdot Pi \cdot l \cdot (\ln (r_a/r_i))^{-1},$$

wobei l die Länge der sich jeweils überlappenden Wandteile von Kolben und Zylinder entspricht, $r_a$ dem radialen Abstand der Zylinderinnenwand von der Zylinderachse, und $r_i$ dem radialen Abstand der Kolbenaußenwand von der Zylinderachse entspricht. $e_o$ ist die absolute Dielektrizitätskonstante und $e_r$ die relative Dielektrizitätskonstante. Bei zunehmender Überlappung von Kolben- und Zylinderwand findet eine Erhöhung der Kapazität, bei abnehmender Überlappung eine Erniedrigung der Kapazität des Zylinderkondensators statt. Somit sind Richtung und Größe der Hubbewegung alleine durch die berührungslose Messung der Kapazitätsänderung des Zylinderkondensators möglich. Die als Dielektrikum für den Kondensator wirkende Rollmembran aus gummielastomerem Material (z.B. unter dem Handelsnamen Viton der Firma Du Pont bekannt) besitzen eine relative Dielektrizitätskonstante $e_r$ von etwa 2, wodurch eine noch genauere und empfindlichere Kapazitätsmessung möglich ist.

Die als Dielektrikum abrollende Rollmembran kann sich je nach konstruktiver Ausführungsform ihrer Einspannung in die Kolben-Zylindereinheit entsprechend ihrer zurückgelegten Hubstrecke mehr oder weniger weit übereinander falten. Dadurch wird zwar die relative Dielektrizitätskonstante wegen der daraus resultierenden Materialverdickung weiter verändert; diese Erscheinung kann jedoch durch eine Kalibrierung in ihrer Auswirkung auf das Meßsignal einbezogen werden.

Will man eine Kalibrierung aus diesem Anlaß vermeiden, hat man Sorge dafür zu tragen, daß die Rollmembran zwischen den leitfähigen Kondensatorplatten stets einlagig abrollt. Dies kann beispielsweise dadurch erreicht werden, daß bei einer Kolben-Endlage die Rollmembran hülsenförmig den Kolben umgibt, und in dieser Lage einer ihrer Endränder in der Zylinderwand eingespannt wird, so daß bei maximaler Überlappung der Kondensatorplatten auch das einlagige Dielektrikum vollständig mit der Kondensatorplattenausdehnung übereinstimmt. Bei einer nachfolgenden Hubbewegung der Kolbenwand wird sich die Rollmembran an ihrer feststehenden Einspannstelle von der Kolbenwand ablösen und eine mitwandernde Falte bilden; der auf der Kolbenwand verbleibende Teil der Rollmembran wird dabei jedoch stets in einlagiger Form zwischen den Kondensatorplatten bleiben, bis Kolben und Membran bei maximaler Hubauslenkung ganz aus dem ihnen zugeordneten elektrisch leitfähigen Wandteil der Zylinderinnenwand entfernt sind.

Zur weiteren Erhöhung der relativen Dielektrizitätskonstante $e_r$ kann die Kolbenwand und/oder die Zylinderwand mit einer dielektrischen Folie ausgekleidet sein. Das Material einer solchen Folie kann aus einem Polyamid bestehen (Handelsname Capton der Firma Du Pont), wodurch die gesamte relative Dielektrizitätskonstante $e_r$ um einen weiteren Faktor 3 bis 5 erhöht wird. Weitere, zur Erhöhung der relativen Dielektrizitätskonstante geeignete Materialien sind Silikon und Neopren. Darüberhinaus kann auch die Zylinderinnenwand mit einer dielektrischen Folie ausgekleidet sein und somit die relative Dielektrizitätskonstante insgesamt erhöhen.

Die einfache Kalibrierung des Zylinderkondensators hinsichtlich seiner Kapazität und der zurückgelegten Wegstrecke macht ihn besonders geeignet, um ihn als Stellglied für eine Steuerung der Kolbenzylindereinheit zur Ausführung vorgegebener Hubbewegungen einzusetzen. Dazu wird die Auswerteeinheit mit einem Regler gekoppelt, in dem die für einen oder mehrere Förderhübe erforderlichen Hubstrecken als Sollwerte gespeichert sind. Die Durchführung der Hubbewegungen wird anhand der Kapazitätsmessung kontrolliert und die dazugehörigen Istwerte mit den Sollwerten verglichen. Bei Abweichung der Istwerte vom Sollwert wird der Kolben von der Antriebseinheit in entsprechender Richtung um die notwendige Hubstrecke bewegt, damit der Sollwert genau erreicht wird.

Ein Ausführungsbeispiel der Erfindung wird anhand der schematischen Zeichnung dargestellt und im folgenden näher erläutert.

Es zeigen:

Fig. 1 einen Schnitt durch die Kolben-Zylindereinheit,

Fig. 2 das Blockschaltbild für den elektrischen Schaltkreis zur Auswertung des Meßsignals,

Fig. 3 die Einzelheit Z aus der Figur 1.

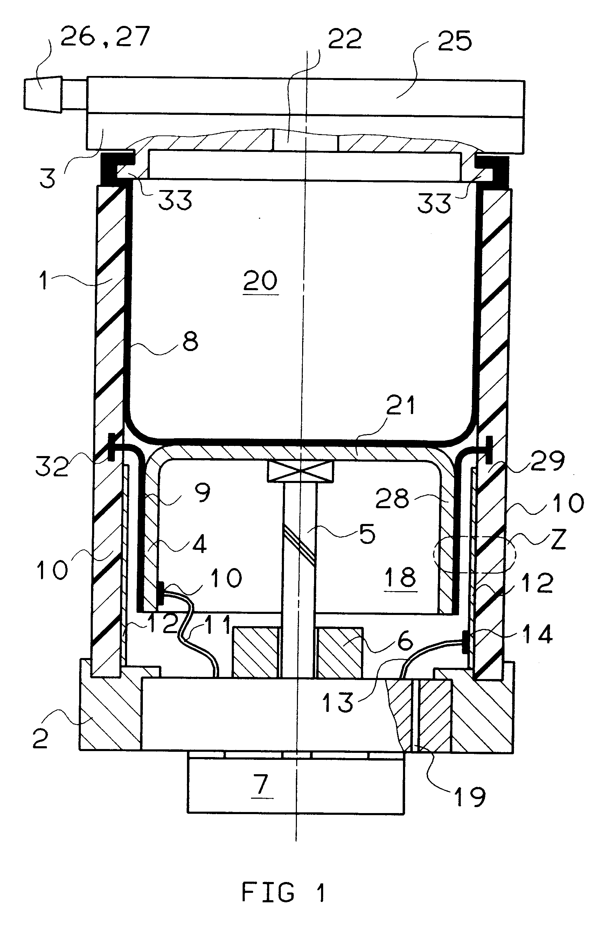

In Fig. 1 ist die Kolben-Zylindereinheit dargestellt, welche einen Zylinder (1) aus Kunststoffmaterial aufweist, dessen Stirnseiten durch eine Grundplatte (2) und eine Anschlußplatte (3) abgeschlossen sind. Im Innern des Zylinders (1) ist ein Kolben (4) auf einer in einem Axiallager (6) geführten Kolbenstange (5) aufgenommen, die mit einer Antriebsvorrichtung (7) verbunden ist. Die Kolbenwand (28) ist gegenüber dem Zylinder (1) mit einer Rollmembran (9) abgedichtet, die um die äußere Wandung des Kolbens (28) als gummielastische Abdeckung gelegt ist. Die Rollmembran (9) ist in Höhe der Stirnplatte (21) des Kolbens (28) in entsprechende Ausschnitte (32) in der Zylinderwand (29) eingeknöpft. Die Rollmembran (9) teilt das innere des Zylinders (1) in eine Vorkammer (18), welche über einen Durchlaß (19) mit der Umgebung verbunden ist, und in eine Arbeitskammer (20), in welcher der durch die Hubbewegung des Kolbens (4) erzeugte Beatmungsdruck hergestellt wird. Die Arbeitskammer (20) ist mit einer Topfrollmembran (8) ausgekleidet, welche in einen Vorsprung (33) der Anschlußplatte (3) eingeknöpft ist und auf der Stirnplatte (21) des Kolbens aufliegt. Somit ist die Arbeitskammer (20) während der gesamten Hubbewegungen des Kolbens (4) mit einem sich den Hubbewegungen anpassenden elastischen Beutel ausgekleidet und sorgt so für eine Trennung zwischen Atemgas führenden Leitungen des nicht dargestellten angeschlossenen Beatmungsgerätes einerseits, und den Antriebsteilen der Kolbenzylindereinheit andererseits. Das während des Arbeitshubes verdrängte Atemgas in der Arbeitskammer (20) kann durch den an der Anschlußplatte (3) angebrachten Anschlußstutzen (22) zu einem Atemsystem (25) des nicht dargestellten Beatmungsgerätes fließen. Das Atemsystem (25) hat Anschlüsse (26, 27) für eine ebenfalls nicht dargestellte Einatemleitung und Ausatemleitung, über die die Atemgasversorgung erfolgt. Ein Teil der inneren Zylinderwand (29) ist mit einem elektrisch leitfähigen Belag in Form einer Beschichtung (12) ausgekleidet, und zwar mindestens soweit, daß sie sich parallel zur Kolbenwand (28) in der in der Figur 1 dargestellten Endstellung des Kolbens (4) erstreckt. An der elektrisch leitenden Beschichtung (12) ist ein elektrischer Kontakt (14) mit einer zugehörigen Anschlußleitung (13) angebracht, durch welche die Beschichtung (12) über die Antriebsvorrichtung (7) mit elektrischer Energie versorgt wird. In gleicher Weise ist der aus elektrisch leitfähigem Material hergestellte Kolben (4) mit einem Anschluß (10) und einer Anschlußleitung (11) versehen. In der dargestellten Endstellung des Kolbens (4) erfährt die Arbeitskammer (20) ihre maximale Ausdehnung. Die Kolbenwand (28) und die mit der Beschichtung (12) versehene Zylinderwand (29) bilden die Flächen eines Zylinder-Kondensators, dessen Kapazität durch den radialen Abstand der Beschichtung (12) und Zylinderwand (28) einerseits und durch die sich überlappenden Wandteilen der Kolbenwand (28) und Zylinderwand (29) andererseits bestimmt ist. Einen weiteren Einfluß auf die Zylinderkapazität besitzt die als Dielektrikum zwischen der Beschichtung (12) und Zylinderwand (28) einlagig ausgebreitete Rollmembran (9).

Beim Betrieb der Kolbenzylindereinheit führt der Kolben (4), angetrieben durch die Kolbenstange (5), Hubbewegungen aus, infolge deren mit fortschreitender Hubbewegung in Richtung der Arbeitskammer (20) die Rollmembran (9) von der Kolbenwand (28) losgelöst wird und sich der überdeckende Flächenanteil von Kolbenwand (28) einerseits und der Beschichtung (12) andererseits verringert. Wegen dieser Verringerung der sich überlappenden Flächenanteile verringert sich die Kapazität des Zylinder-Kondensators linear bis zu dem Punkt, wo sie den minimalen Wert erreicht, wenn der Kolben (4) in seiner maximalen Hublänge in Richtung Arbeitskammer (20) ausgefahren ist. Der Kolben (4) befindet sich dann in seiner nicht dargestellten oberen Totpunktlage, die durch die minimale, konstante Restkapazität festgelegt ist. Bei der Rückwärtsbewegung des Kolbens (4) zu seiner in der Figur 1 dargestellten unteren Totpunktlage nimmt die Kapazität des Kondensators in dem Maße wieder zu, wie sich mit beginnender Überschneidung der Rollmembran (9) mit der Beschichtung (12) als Kondensatorflächen der sich überlappende Anteil vergrößert. In seiner dargestellten unteren Totpunktlage besitzt der so gebildete Zylinderkondensator seine größtmögliche Kapazität. Das während der gesamten Hubbewegung gewünschte einlagige Abrollen der Membran wird dadurch erreicht, daß die Befestigungsstellen als Einknüpfungen (32) für die Rollmembran (9) einseitig jenseits des leitenden Bereiches der Zylinderwand (29), wie er durch die Beschichtung (12) festgelegt ist, vorgesehen sind. Dabei befindet sich der Kolben (4) in seiner unteren Totpunktlage, um den Befestigungsort für die Rollmembran (9) festzulegen, damit eine maximale Überlappung zwischen Kolbenwand (28) und Beschichtung (12) erzielt wird.

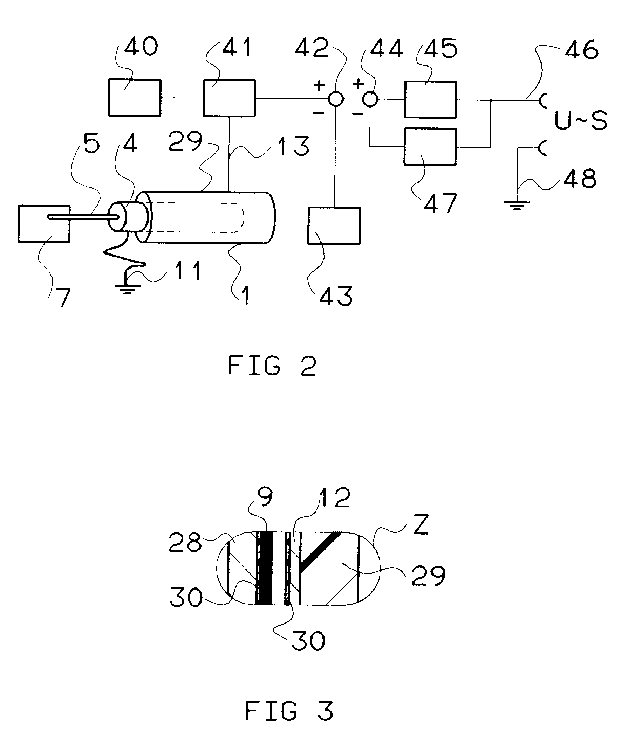

Das in Figur 2 dargestellte Blockschaltbild zeigt den Zylinder (1) mit dem schematisch dargestellten, in dem Zylinder (1) geführten Kolben (4), der über die Antriebseinheit (7) mittels der Kolbenstange (5) bewegt wird. Die elektrisch leitfähige Kolbenwand (28) ist über die Anschlußleitung (11) an Masse gelegt. Die in Figur 1 alle der Antriebseinheit (7) zugeordneten elektronischen Steuerbauteile sind in der Figur 2 einzeln aufgeführt. Von einem mit einer Frequenz von etwa 10 kHz arbeitenden Multivibrator (40) wird die hochfrequente Wechselspannung an einen monostabilen Multivibrator (Monoflop) weitergeleitet, dessen Signalimpulse von etwa 20 kHz über die Anschlußleitung (13) an den leitfähigen Teil der Zylinderwand (29) des Zylinders (1) weitergegeben werden. Kolben (4) und Zylinder (1) bilden somit die veränderliche Kapazität eines Zeitgliedes, dessen Impulsdauern sich mit verändernder Kapazität im Größenordnungsbereich von etwa 10 bis 100 Mikrosekunden verändern. Die von dem Monoflop (41) abgegebenen, von der Kapazität des Zylinderkondensators (4, 29) abhängenden Signale werden einem Operationsverstärker (42) zugeführt, durch den mit Hilfe eines Pulslängenkorrekturgliedes (43) die Impulslängen auf eine solche Größe verändert werden, wie sie sich allein aus der sich verändernden Kapazität des Zylinder-Kondensators (4, 29) ergeben. Das Pulslängenkorrekturglied (43) hat die Aufgabe, die schaltungsbedingte Kapazität aus dem übrigen elektrischen Schaltkreis herauszufiltern und sie von dem kapazitätsabhängigen Anteil von der Kolbenzylindereinheit auszusondern. Das so korrigierte Signal wird einem weiteren Operationsverstärker (44) zugeführt, dessen Ausgang über ein Aktivfilter (45) an dem Signalabgriff (46) anliegt. Parallel zu dem Aktivfilter (45) ist eine Gleichspannungsunterdrückung (47) zwischen Signalabgriff (46) und invertierendem Eingang des Operationsverstärkers (44) geschaltet. Somit liegt an dem Signalabgriff (46) ein Spannungssignal an, das durch die korrigierten Impulse des Multivibrators (40) in Abhängigkeit von der Lage des Kolbens (4) relativ zu der Zylinderwand (29) gebildet ist. Der Signalabgriff (46) liegt über seine Potentialleitung (48) ebenfalls an Masse.

Je nach Lage des Kolbens (4) im Zylinder (1) wird wegen der sich verändernden Kapazität des Zylinder-Kondensators, gebildet aus dem sich überlappenden Teil der der durch die Beschichtung (12) leitfähig gemachten Zylinderwand (29) einerseits und der mit der dielektrischen Rollmembran (9) ausgekleideten Kolbenwand (28) andererseits, die Impulslänge am Signalabgriff (46) in recht gutem linearen Zusammenhang verändert, so daß sich nach einmal anfänglich erfolgter Kalibrierung, beispielsweise mit Hilfe eines Schiebepotentiometers, welches an die Kolbenstange (5) angeschlossen ist und durch das sich das Signal am Signalabgriff (46) in Weglänge kalibrieren läßt, eine eindeutige Zuordnung zwischen Signallänge und Wegauslenkung ergibt.

In Figur 3 ist die Einzelheit Z aus Figur 1 deutlicher dargestellt. Die Einzelheit Z betrifft einen Ausschnitt aus demjenigen Teil der Kolben-Zylindereinheit, welche den Plattenkondensator bildet. Dabei ist die Kolbenwand (28) mit einer dielektrischen Folie (30) ausgekleidet, über welche die Rollmembran (9) gespannt ist. Ihr gegenüber befindet sich die elektrisch leitfähige Beschichtung (12) auf der aus nicht leitfähigem Kunststoff hergestellten Zylinderwand (29). Die Beschichtung (12) trägt ebenfalls eine dielektrische Folie (30).

**Patentansprüche**

1. Kolben-Zylindereinheit als über eine Antriebseinheit ansteuerbare Fördervorrichtung für das Atemgas in einem Beatmungsgerät, wobei der Kolben relativ zu dem Zylinder bewegbar, seine Bewegung von einem Wegaufnehmer erfaßbar und eine zwischen Kolbenwand und Zylinderwand abrollende Rollmembran als Dichtung vorgesehen ist, dadurch gekennzeichnet, daß zumindest ein Teil der Zylinderwand (29) einerseits und der Kolbenwand (28) andererseits als Platten eines elektrischen Zylinder-Kondensators ausgebildet sind, zwischen denen die Rollmembran als Dielektrikum (9) die sich jeweils überlappenden elektrisch-leitenden Wandteile (28, 12) von Kolben (4) und Zylinder (1) abdeckt, und daß die bei der Hubbewegung sich verändernde Kapazität des Kondensators (9, 12, 28) als Wegmessung für die Hubstrecke des Kolbens (4) durch eine Auswerteeinheit (40, 41, 42, 44, 45, 46) erfaßbar ist.

2. Kolbenzylindereinheit nach Anspruch 1, dadurch gekennzeichnet, daß die Rollmembran an der Zylinderwand (29) derart eingespannt ist, daß sie stets als einlagige Zwischenschicht (9) zwischen Kolbenwand (28) und elektrisch-leitfähiger Zylinderwand (12) als Kondensatorplatten abrollt.

3. Kolbenzylindereinheit nach Anspruch 1 oder 2, dadurch gekennzeichnet, daß zur Erhöhung der relativen Dielektrizitätskonstanten $e_r$ des Dielektrikums (9) die Kolbenwand (28) und/oder die Zylinderwand (29) mit einer dielektrischen Folie (30) ausgekleidet ist.

FIG 1

FIG 2

FIG 3

Europäisches
Patentamt

**EUROPÄISCHER RECHERCHENBERICHT**

Nummer der Anmeldung

EP    92 10 9021

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 342 435 (DRÄGERWERK AG)<br>* Zusammenfassung; Abbildungen *<br>--- | 1 | A61M16/00<br>F04B51/00<br>G01F3/16<br>G01D5/24<br>F15B15/28 |
| A | US-A-4 010 761 (TIPPLE)<br>* Zusammenfassung; Abbildung 3 *<br>--- | 1 | |
| A | WO-A-9 010 197 (ROBERT BOSCH GMBH)<br>* Zusammenfassung; Abbildung 1 *<br>--- | 1 | |
| A | FR-A-2 482 723 (LESLIE HARTRIDGE LIMITED)<br>* Seite 3, Zeile 20 - Seite 4, Zeile 7; Abbildung 1 *<br>--- | 1 | |
| A,P | PATENT ABSTRACTS OF JAPAN<br>vol. 16, no. 193 (P-1349)11. Mai 1992<br>& JP-A-40 27 863 ( YANMAR AGRICULT EQUIP CO LTD ) 30. Januar 1992<br>* Zusammenfassung *<br>--- | 1 | |
| A | DE-A-3 518 858 (NISSAN MOTOR CO., LTD.)<br>* Zusammenfassung; Abbildungen 1-3 *<br>* Seite 8, Zeile 31 - Seite 9, Zeile 15 *<br>* Seite 13, Zeile 16 - Seite 14, Zeile 2 *<br><br>----- | 1 | **RECHERCHIERTE SACHGEBIETE (Int. Cl.5)**<br><br>A61M<br>F04B<br>G01F<br>B60G |

Der vorliegende Recherchenbericht wurde für alle Patentansprüche erstellt

| Recherchenort | Abschlußdatum der Recherche | Prüfer |
|---|---|---|
| DEN HAAG | 30 SEPTEMBER 1992 | ZEINSTRA H. |